# EUROPEAN PATENT SPECIFICATION

(11) **EP 2 411 023 B1**
(45) Date of publication and mention of the grant of the patent: **29.11.2017**
(21) Application number: 10755333.1
(22) Date of filing: 29.03.2010
(51) Int. Cl.: A61K 35/32, A61L 31/16

(54) **METHOD OF TISSUE REPAIR**
GEWEBEREPARATURVERFAHREN
PROCÉDÉ DE RÉPARATION DE TISSU

(30) Priority: 27.03.2009 AU 2009901325
(43) Date of publication of application: 01.02.2012
(73) Proprietor: Orthocell Limited, Murdoch WA 6150 (AU)
(72) Inventor: ZHENG, Ming Hao, City Beach Western Australia 6015 (AU)
(74) Representative: Ström & Gulliksson AB
(86) International application number: PCT/AU2010/000360
(87) International publication number: WO 2010/108237

(56) References cited:
- WO-A2-02/067856
- WO-A2-03/087303
- WO-A2-2005/025493
- US-A1- 2002 155 096
- BEHRENS P ET AL: "Matrix-associated autologous chondrocyte transplantation/implantation (MACT/MACI)-5-year follow-up", THE KNEE, ELSEVIER, AMSTERDAM, NL, vol. 13, no. 3, 1 June 2006 (2006-06-01), pages 194-202, XP027992130, ISSN: 0968-0160 [retrieved on 2006-06-01]
- MARLOVITS S ET AL: "Cartilage repair: Generations of autologous chondrocyte transplantation", EUROPEAN JOURNAL OF RADIOLOGY, ELSEVIER SCIENCE, NL, vol. 57, no. 1, 1 January 2006 (2006-01-01), pages 24-31, XP028004715, ISSN: 0720-048X, DOI: 10.1016/J.EJRAD.2005.08.009 [retrieved on 2006-01-01]
- BRYAN, N ET AL.: 'Derivation and performance of an entirely autologous injectable hydrogel delivery system for cell-based therapies' BIOMATERIALS vol. 30, 2009, pages 180 - 188, XP025609208
- RINGE, J ET AL.: 'Tissue engineering in the rheumatic diseases' ARTHRITIS RESEARCH & THERAPY, [Online] vol. 11, no. 1, 2009, pages 1 - 11, XP055095917 Retrieved from the Internet: <URL:http://arthritis-research.com/content/ 11/1/211> [retrieved on 2009-01-30]

## Description

### FIELD

The present invention relates to a method of producing an implantable matrix for repairing tissue. More specifically, the present invention relates to methods of using autologous mammalian chondrocyte cells and an implantable support for the producing an implantable matrix for the repair of tissue defects.

### INTRODUCTION

Increasingly there is a demand for new treatment strategies for repairing tissue damage due to the limitations of conventional treatment regimes and an aging population. Currently, cell-based therapies represent the state of the art for treating defects in tissues and organs. These therapies involve introducing progenitor cells, preferably stem cells, into the defect site, which boosts endogenous cell populations and increases the rate of tissue regeneration and repair. These cells are often autologous in nature, isolated from the patient requiring treatment and expanded *in vitro* before being returned to the patient at the site of the defect.

After the explanted cells are expanded, it is common practice for the cells to be cultured for a further 4 to 10 days on a support or scaffold. The cell/scaffold composition is then implanted at the site of the tissue defect. A scaffold is used in conjunction with autologous cells for three main reasons: (1) to provide an environment that mirrors the extracellular matrix, which is thought to be conducive to cell growth; (2) to encourage the formation of tissue architecture; and (3) to provide mechanical strength to the newly forming tissue once implanted.

However, there are a number of problems with the current methods. Firstly, it is widely known that cells cultured *in vitro* for long periods of time differentiate, which decreases the ability of the cells to proliferate and repair tissue *in vivo.* Secondly, the culturing of cells *in vitro* exposes the cells to foreign materials that may contain contaminating particles (such as viruses or bacteria) or chemicals. These contaminates, if not detected before implantation, have the potential to cause significant disease and morbidity. Further, the risk of the cells becoming contaminated increases with the length of time cells are cultured. Lastly, cells cultured on scaffolds rarely penetrate more than 500µm from the external surface due to lack of nutrients and oxygen. As such, despite efforts to encourage the formation of tissue architecture, full-thickness tissues cannot be formed *in vitro.*

Accordingly, there is a need in the art to identify better ways of utilising cells and scaffolds in the repair of tissues.

### SUMMARY

The inventors have developed a novel approach to the repair of tissues comprising the application of cells to an implantable support less than 1 hour 20 minutes before implantation.

Accordingly, in a first aspect the present invention provides a method of producing an implantable matrix comprising the steps of: (a) providing an implantable support comprising cross-linked or uncross-linked collagen, and a sample of autologous mammalian chondrocyte cells; and (b) applying said sample of autologous mammalian chondrocyte cells to the surface of said support to produce said implantable matrix; wherein the cells are allowed to incubate for between about 20 minutes to 1 hour 20 minutes before implantation of the matrix; wherein said implantable support comprises a membrane.

It is important that the cells are not cultured *in vitro* with the implantable support before implantation, but are merely allowed sufficient time to adhere to the implantable support before implantation.

It will be appreciated that the tissue in need of repair may be any tissue found in a mammalian animal, including but not limited to epithelium, connective tissue or muscle. In some embodiments the tissue is cartilage. Similarly, it will be understood that the cells used in the methods of the invention as described herein can be isolated from any tissue found in a mammalian animal.

The cells may be isolated from any mammalian animal including, but not limited to a sheep, a cow, a pig, a horse, a dog, a cat or a human. In other embodiments, the cells are isolated from a human. In still other embodiments, the cells are isolated from the animal subject in need of treatment.
In one embodiment the mammalian chondrocyte cells are human chondrocyte cells.

The implantable support may be any type of implantable support used for repairing tissues. The implantable support comprises a membrane.

In some embodiments, the method further comprises the step of coating the implantable matrix after the mammalian chondrocyte cells have adhered with a cell sealant prior to implantation. The cell sealant may be any surgical tissue adhesive. In some embodiments, the cell sealant is a fibrin sealant.

It will be appreciated that the purpose of the invention is to implant the matrix comprising an implantable scaffold and adhered cells as soon as the cells have adhered to the support i.e. the matrix is not cultured *in vitro* before implantation. Accordingly, the cells may be applied to the support up to about 1 hour 20 minutes before the implantable matrix is implanted. For example, the cells may be applied to the support between about 30 minutes and about 1 hour and 10 minutes before implantation; between about 30 minutes and about 1 hour before implantation; or between about 40 minutes and about 50 minutes before implantation. In some embodiments, the cells are applied to the support about 40 minutes before implantation.

In a specific embodiment, the implantable support is heated to between 35°C and 37°C before the mammalian chondrocyte cells are applied.

It will be appreciated by those skilled in the art that the purpose of the short (incubation) time between applying or seeding the implantable support with cells and implanting the matrix produced (less than 2 hours) is to reduce the cell death of the primary cells that typically accompanies prolong culture.

Thus, a method of increasing the viability of cells for implantation comprises applying a sample of cells to an implantable support and implanting said support within 2 hours of the cells having been applied thereto.

The implanted cells may have a viability of greater than 90% or greater than 95%. The implanted cells may have a viability of greater than 99% immediately prior to implantation.

It will be appreciated that because the cells of the present invention have a high viability the cells will also have a lower expression of apoptosis indicators. In some embodiments, the indicators of apoptosis are selected from the group consisting of MMP-1, MMP-9, MMP-13, ADAMTS-4, IL-1, c-fos, c-jun, Oct3/4 and Sox2.
In some embodiments, the mammalian chondrocyte cells are suspended in the subjects own serum before being applied to the implantable support.

### BRIEF DESCRIPTION OF THE FIGURES

Figure 1: Comparison of gene expression in human cells grown with (dark bars) and without (light bars) a collagen scaffold (* = p<0.05).
Figure 2: Time dependent cell adhesion on a collagen scaffold (* = p<0.05).

### DETAILED DESCRIPTION OF THE PREFERRED EMBODIMENTS

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting which will be limited only by the appended claims. The publications mentioned herein are cited for the purpose of describing and disclosing the protocols and reagents which are reported in the publications and which might be used in connection with the invention. Nothing herein is to be construed as an admission that the invention is not entitled to antedate such disclosure by virtue of prior invention.
The practice of the present invention will employ, unless otherwise indicated, conventional techniques of cell culture, cell biology and orthopedic surgery, which are within the skill of the art. Such techniques are described in the literature. See, for example, Coligan et al., 1999 "Current protocols in Protein Science" Volume I and II (John Wiley & Sons Inc.); Ross et al., 1995 "Histology: Text and Atlas", 3rd Ed., (Williams & Wilkins); Kruse & Patterson (eds.) 1977 "Tissue Culture" (Academic Press); Canale (ed.) 2003 "Campbell's Operative Orthopaedics" 10th ed. (St. Louis, Mo. : MD Consult LLC);and Alberts et al. 2000 "Molecular Biology of the Cell" (Garland Science).
It must be noted that as used herein and in the appended claims, the singular forms "a," "an," and "the" include plural reference unless the context clearly dictates otherwise. Thus, for example, a reference to "a cell" includes a plurality of such cells, and a reference to "an implantable support" is a reference to one or more implantable supports, and so forth. Unless defined otherwise, all technical and scientific terms used herein have the same meanings as commonly understood by one of ordinary skill in the art to which this invention belongs.

Although any materials and methods similar or equivalent to those described herein can be used to practice or test the present invention, the preferred materials and methods are now described.

The term "tissue", as used herein, refers to a collection of mammalian cells that are grouped together and specialise in performing a particular function. The cells may be of the same type, for example nervous tissue comprising only nerve cells, or many different types, for example connective tissue comprising cells such as fibroblasts and adipose cells, as well as transient populations of cells such as mast cells, macrophages, monocytes, lymphocytes, plasma cells and eosinophils. Epithelium (epithelia) tissue, connective tissue and muscle tissue comprise cells that have phenotypic characteristics in common across species. For example, epithelia from all mammalian species generally comprise a single layer of cells held together by occluding junctions called tight junctions. More importantly, all cells within epithelia from any mammalian species have similar growth characteristics.
Connective tissue comprises a number of cells, which are common to all mammalian species. For example, connective tissue cells include blood cells (erythrocytes and leukocytes (polymorphonuclear leukocytes, eosinophils, basophils, monocytes and lymphocytes)), megakaryocytes, fibroblasts (including chondroblasts and osteoblasts), macrophages, mast cells, plasma cells, adipose cells and osteoclasts. Examples of connective tissues are tendons, cartilage and ligaments. Bone and blood are examples of specialized connective tissues.
Muscle tissue also comprises cells (fibres) that have a common ancestry and therefore morphology, physiology and phenotypic characteristics

Accordingly, as used herein, the term "tissue" refers to any collection of cells within a mammalian animal that requires repair.

Soft tissue, as used herein, refers generally to extraskeletal structures found throughout the body and includes but is not limited to cartilage tissue, meniscal tissue, ligament tissue, tendon tissue, intervertebral disc tissue, periodontal tissue, skin tissue, vascular tissue, muscle tissue, fascia tissue, periosteal tissue, ocular tissue, pericardial tissue, lung tissue, synovial tissue, nerve tissue, kidney tissue, bone marrow, **urogenital** tissue, intestinal tissue, liver tissue, pancreas tissue, spleen tissue, or adipose tissue, and combinations thereof.

Soft tissue condition (or injury or disease) is an inclusive term encompassing acute and chronic conditions, disorders or diseases of soft tissue. For example, the term encompasses conditions caused by disease or trauma or failure of the tissue to develop normally. Examples of soft tissue conditions include but are not limited to hernias, damage to the pelvic floor, tear or rupture of a tendon or ligament, skin wounds (e.g., scars, traumatic wounds, ischemic wounds, diabetic wounds, severe burns, skin ulcers (e.g., decubitus (pressure) ulcers, venous ulcers, and diabetic ulcers), and surgical wounds such as those associated with the excision of skin cancers); vascular conditions (e.g., vascular disease such as peripheral arterial disease, abdominal aortic aneurysm, carotid disease, and venous disease; vascular injury, improper vascular development); and muscle diseases (e.g., congenital myopathies; myasthenia gravis; inflammatory, neurogenic, and myogenic muscle diseases; and muscular dystrophies such as Duchenne muscular dystrophy, Becker muscular dystrophy, myotonic dystrophy, limb-girdle-muscular dystrophy, facioscapulohumeral muscular dystrophy, congenital muscular dystrophies, oculopharyngeal muscular dystrophy, distal muscular dystrophy, and Emery-Dreifuss muscular dystrophy).

The present invention is directed towards a method for producing an implantable matrix for the repair of cartilage. The term "cartilage" refers to a type of connective tissue that contains chondrocytes or chondrocyte-like cells (having many, but not all characteristics of chondrocytes) and intercellular material (e.g., Types I, II, IX and XI collagen), proteoglycans (e.g., chondroitin sulphate, keratin sulphate, and dermatan sulphate proteoglycans) and other proteins. Cartilage includes articular and non-articular cartilage.

"Articular cartilage," also referred to as hyaline cartilage, refers to an avascular, non-mineralized connective tissue, which covers the articulating surfaces of bones in joints and serves as a friction reducing interface between two opposing bone surfaces. Articular cartilage allows movement in joints without direct bone-to-bone contact. Articular cartilage has no tendency to ossification. The cartilage surface appears smooth and pearly macroscopically, and is finely granular under high power magnification. Articular cartilage derives nutrients partly from the vessels of the neighbouring synovial membrane and partly from the vessels of the bone it covers. Articular cartilage is associated with the presence of Type II and Type IX collagen and various well-characterized proteoglycans, and with the absence of Type X collagen, which is associated with endochondral bone formation. For a detailed description of articular cartilage microstructure, see, for example, Aydelotte and Kuettner, Conn. Tiss. Res., 18, p. 205 (1988); Zanetti et al., J. Cell Biol., 101, p. 53 (1985); and Poole et al., J. Anat., 138, p. 13 (1984).

"Non-articular cartilage" refers to cartilage that does not cover articulating surfaces and includes fibrocartilage (including interarticular fibrocartilage, fibrocartilaginous disc, connecting fibrocartilage and circumferential fibrocartilage) and elastic cartilage. In fibrocartilage, the micropolysaccharide network is interlaced with prominent collagen bundles, and the chondrocytes are more widely scattered than in hyaline or articular cartilage. Interarticular fibrocartilage is found in joints which are exposed to concussion and subject to frequent movement, e.g., the meniscus of the knee. Examples of such joints include but are not limited to the temporo-mandibular, stemo-clavicular, acromio-clavicular, wrist and knee joints. Secondary cartilaginous joints are formed by discs of fibrocartilage. Such fibrocartilaginous discs, which adhere closely to both of the opposed surfaces, are composed of concentric rings of fibrous tissue, with cartilaginous laminae interposed. An example of such fibrocartilaginous disc is the intervertebral disc of the spine. Connecting fibrocartilage is interposed between the bony surfaces of those joints, which allow for slight mobility as between the bodies of the vertebrae and between the pubic bones. Circumferential fibrocartilage surrounds the margin of some of the articular cavities, such as the cotyloid cavity of the hip and the glenoid cavity of the shoulder.

The terms "repairing" or "repair" or grammatical equivalents thereof are used herein to cover the repair of a tissue defect in a mammalian animal, preferably a human. "Repair" refers to the formation of new tissue sufficient to at least partially fill a void or structural discontinuity at a tissue defect site. Repair does not however, mean or otherwise necessitate a process of complete healing or a treatment, which is 100% effective at restoring a tissue defect to its pre-defect physiological/structural/mechanical state.
The term "tissue defect" or "tissue defect site" refers to a disruption of epithelium, connective or muscle tissue. A tissue defect results in a tissue performing at a suboptimal level or being in a suboptimal condition. For example, a tissue defect may be a partial thickness or full thickness tear in a tendon or the result of local cell death due to an infarct in heart muscle. A tissue defect can assume the configuration of a "void", which is understood to mean a three-dimensional defect such as, for example, a gap, cavity, hole or other substantial disruption in the structural integrity of the epithelium, connective or muscle tissue. In certain embodiments, the tissue defect is such that it is incapable of endogenous or spontaneous repair. A tissue defect can be the result of accident, disease, and/or surgical manipulation. For example, cartilage defects may be the result of trauma to a joint such as a displacement of torn meniscus tissue into the joint. Tissue defects may be also be the result of degenerative diseases such as osteoarthritis.
At the most basic level, the implantable matrix produced by the method according to the present invention may be used for the implantation of cells at the site of the tissue defect. These cells boost endogenous cell populations and increase the rate of tissue regeneration and repair.

The cells of the present invention may be isolated from a tissue in a variety of ways, all which are known to one skilled in the art. In some embodiments, the cells can be isolated from a biopsy material by conventional methods. As described in more detail below, in some embodiments, the cells are isolated by enzymatic digestion.

According to the invention, the tissue containing the cells of interest are be isolated from any mammalian animal including, but not limited to a sheep, a cow, a pig, a dog, a cat, a horse or a human. In other embodiments, the tissue is isolated from a human. Preferably, the tissue is isolated from the same species of mammalian animal that has the tissue defect.

According to the invention, the tissue is "autologous", i.e. isolated from the body of the subject in need of treatment. For example, a mammalian animal with a cartilage tear in their knee can have a biopsy taken from any cartilage in their body, for example the upper outer medial aspect of the femoral condyles.

The cells may be obtained from biopsy material by appropriate treatment of the tissue that is to serve as the source of the cells. Techniques for treatment of tissue to isolate cells are known to those skilled in the art see, for example, Freshney "Culture of Animal Cells. A Manual of Basic Technique" 2nd ed. (A. R. Liss Inc.). For example, the tissue or organ can be mechanically disrupted and/or treated with digestive enzymes or chelating agents to weaken the interactions between cells making it possible to obtain a suspension of individual cells. Typically the method will include a combination of mechanical disruption, enzyme treatment and chelating agents. In one technique the tissue is minced and treated simultaneously or subsequently with any of a number of digestive enzymes either alone or in combination. Examples of enzymes useful in dissociating cells include, but are not limited to, trypsin, chymotrypsin, collagenase, elastase, hyaluronidase, DNase, pronase, dispase, and the like. In some embodiments, enzyme compositions containing an aqueous mixture of collagenase having an activity of about 43 nkat/ml to about 51 nkat/ml, and chymopapain having an activity of about 0.22 nkat/ml to about 0.44 nkat/ml are used for dissociating cells, such as described in US Patent No. 5,422,261. Mechanical disruption can also be accomplished by, for example, the use of blenders, sieves, homogenizers, pressure cells, and the like.

The resulting suspension of cells and cell clusters can be further divided into populations of substantially homogenous cell types. This can be accomplished using standard techniques for cell separation including, for example, positive selection methods (e.g., clonal expansion and selection of specific cell types), negative selection (e.g., lysis of unwanted cells), separation based upon specific gravity in a density solution, differential adherence properties of the cells in the mixed population, fluorescent activated cell sorting (FACS), and the like. Other methods of selection and separation are known in the art see, for example Freshney "Culture of Animal Cells. A Manual of Basic Technique" 2nd ed. (A. R. Liss Inc.).

In some embodiments, the cells are immediately applied to an implantable support once isolated. Accordingly, the biopsy procedure that isolates the cells and the repair procedure that involves the implantation of the isolated cells at the defect site may be performed sequentially in a single surgery.

Alternatively, in other embodiments, the isolated cells are cultured for a short period of time to increase cell numbers before being applied to the implantable support. The reagents and methods employed to culture the cells will, of course, vary depending on the cell type. For example, if the cells are muscle cells the culture medium may comprise Ham's nutrient mixture F-10 with 0.5% chicken embryo extract and either 20% (vol/vol) foetal calf serum or horse serum. Alternatively, if the cells are epithelial cells the culture medium may comprise Dulbecco's Modified Eagle Medium mixed with Ham's F12 medium with about 5% foetal calf serum. However, one skilled in the art would know how to choose the cell culture medium appropriate for the cell type being cultured. In addition, various media additives may be employed as well, including antibiotics, hormones, growth factors, nutritional supplements, vitamins, minerals and the like. Again, a person skilled in the art would know what additives were required to grow a particular cell type.

The period of time the cells are cultured for will also vary. The culture time may be dependent on the type of cells being cultured and the number of cells required, as well as logistical factors such as when the cells are required. However, it is an important aspect of the invention that the cells are not cultured for a period of time long enough to impact on cellular differentiation or cell phenotype. Preferably, the isolated cells are cultured for no more than about 10 days. However, the cells may be cultured for between about 1 day and about 9 days; between about 2 days and about 8 days; between about 3 days and about 7 days; between about 4 days and about 6 days; or about 5 days. In some embodiments, the cells are cultured for about 4 days.

It is also an important aspect of the invention that the cells are not cultured with any type of implantable support, which induces cellular differentiation and changes to cell phenotype.

The term "implantable support" refers to any matrix or scaffold that is suitable for use in cell implantation with or without an adhesive. According to the present invention, the implantable support comprises a membrane.

The implantable support comprises cross-linked or uncross-linked collagen. In some embodiments the implantable support is made of a semi-permeable material which may include cross-linked or uncross-linked collagen, preferably type I in combination with type III, or type II. The implantable support may also include polypeptides or proteins obtained from natural sources or by synthesis, such as hyaluronic acid, small intestine submucosa (SIS), peritoneum, pericardium, polylactic acids and related acids, blood (i.e., which is a circulating tissue including a fluid portion (plasma) with suspended formed elements (red blood cells, white blood cells, platelets), or other material which is bioresorbable. Bioabsorbable polymers, such as elastin, fibrin, laminin and fibronectin are also useful in the present invention. Support matrix or scaffold materials as described in US Publication No. 20020173806, herein incorporated by reference in its entirety, are also useful in the present invention.
The implantable support is preferably initially (i.e., before contact with the cells to be implanted) free of intact cells and is preferably resorbable within the mammalian animal. The implantable support may have one or several surfaces, such as a porous surface, a dense surface, or a combination of both. The implantable support may also include semi-permeable, impermeable, or fully permeable surfaces. Support scaffolds having a porous surface are described, for example, in US Pat. No. 6,569,172.

The implantable support may be autologous or allogeneic. In some embodiments, a suitable autologous implantable support is formed from blood, as exemplified in US Pat. No. 6,368,298, issued to Berretta, et al. on Apr. 9, 2002.

A suitable implantable support may be a solid, semi-solid, gel, or gel-like scaffold characterized by being able to hold a stable form for a period of time to enable the adherence and/or growth of cells thereon. Examples of suitable implantable supports are disclosed in US Publication No. 20020173806.

Additional examples of suitable implantable supports for growth of tenocytes include Vitrogen™, a collagen-containing solution which gels to form a cell-populated matrix, and the connective-tissue scaffolds of Hwang (US patent application no. 20040267362), Kladaki et al (US patent application no. 20050177249), Giannetti (US patent application no. 20040037812) and Binette et al (US patent application no. 20040078077).

The implantable support can be cut or formed into any regular or irregular shape. In some embodiments, the implantable support can be cut to correspond to the shape of the tear. The implantable support can be flat, round and/or cylindrical in shape. The shape of the implantable support can also be moulded to fit the shape of a particular defect in need of repair. If the implantable support is a fibrous material, or has the characteristics of a fibre, the support matrix can be woven into a desired shape. Alternatively, the bioscaffold can be a gel, gel-like, or non-woven material.
In some embodiments the implantable support is comprised **of** porcine-derived type I/III collagen, for example, ACI Matrix™. In other embodiments the implantable support is comprised of small intestinal submucosa, for example Restore™.
The isolated sample of cells is applied to the implantable support to form an "implantable matrix". Unlike conventional methods, the method of the present invention requires that the sample of cells be applied to the implantable support for less than 2 hours before the implantable matrix is to be used. As discussed elsewhere, conventional methods require that cells are cultured with an implantable support for several days before implantation. However, the inventors of the present invention have found that 100% adhesion of cells to an implantable support can be achieved in less than 2 hours and that cells cultured with an implantable support, for example a collagen scaffold, have a lower viability than cells cultured without an implantable support.
Accordingly, a method of increasing the viability of cells for implantation is by contacting cells for implantation with an implantable support less than 1 hour and 20 minutes before the cells and support are to be implanted.
Methods of measuring the viability of a cell population are well known to those in the art. For example, the expression of apoptosis indicators may be measured. The term "apoptosis indicator", as used herein, refers to genes or corresponding products, that are expressed when a cell is undergoing apoptosis. As such, a population of cells with high viability will have less expression of apoptosis indicators than a population of cells with a lower viability. Examples of apoptosis indicators include matrix metallo-proteases (e.g. MMP-1, MMP-9, MMP-13), ADAMTS-4, IL-1, c-fos, c-jun, Oct3/4 and Sox2.

Other prior art methods, such as that disclosed in US patent application No. 2002/0155096 (hereinafter "US 2002/0155096"), describe the application of stem cells to a scaffold directly or immediately before implantation. However, the timing used in US 2002/0155096 is due to the use of an alginate matrix, which becomes weak if soaked in the cell solution for too long (Example 5, page 7). In contrast, the present method requires cells be applied to the support around at least 20 minutes before implantation to allow sufficient numbers of cells to adhere to the support. Application of the cells to the support less than about 7 minutes before implantation may result in large numbers of cells being lost from the support upon implantation, which may result in suboptimal tissue repair.

Before the implantable matrix is implanted the matrix may be coated with a cell sealant. Cell sealants enable the cell seeded scaffold to attach to an area being treated such as a tissue defect. Cell sealant could also promote the proliferation and migration of cell into the defect area (see, for example, Kirilak & Zheng et al., 2006, Int. J. Mol. Med., 17(4):551-8, herein incorporated by reference). Cell sealants may be a variety of natural and synthetic agents and include fibrin sealants, marine adhesives, collagen fleece, gelatine sponges, cyanoacrylate derivatives and glucose polymers including dextran derivatives. The cell sealant used to the present invention may vary depending on the tissue being repaired. For example, the cyanoacrylates are bacteriostatic for many bacteria and, as such, are frequently used in periodontics and oral surgery. Bovine albumin and glutaraldehyde glue (BioGlue; CryoLife, Inc., Kennesaw, Georgia) are authorized for use during surgical repair of acute thoracic aortic dissection. Fibrin sealant, also referred to as "fibrin glue" or "fibrin tissue adhesive," comprised of purified, virus-inactivated human fibrinogen, human thrombin, and sometimes added components, such as virus-inactivated human factor XIII and bovine aprotinin. Fibrin sealants are currently used in a number of surgical specialties, including cardiovascular surgery, thoracic surgery, neurosurgery, plastic and reconstructive surgery, and dental surgery. In some embodiments, the cell sealant is a combination of glucose polymers and polylysine, which enhances cell attachment and reduces bleeding during surgery.
Once assembled, the implantable matrix may be secured in place by any conventional means known to those skilled in the art, e.g. suturing, suture anchors, bone fixation devices and bone or biodegradable polymer screws. In the case when a cell seeded scaffold is required to repair a non-contained defect of articular cartilage, biodegradable screws can be used in conjunction of fibrin glue to secure the attachment of scaffold to the defect.
The compositions as disclosed in the embodiments of the invention may be part of a kit. Typically the kit would also include instructions for use.

The invention will now be further described by way of reference only to the following non-limiting examples. It should be understood, however, that the examples following are illustrative only, and should not be taken in any way as a restriction on the generality of the invention described above.

### EXAMPLE 1 TREATMENT OF CARTILAGE DEFECT USING AUTOLOGOUS CELLS WITH IMPLANTABLE SUPPORT

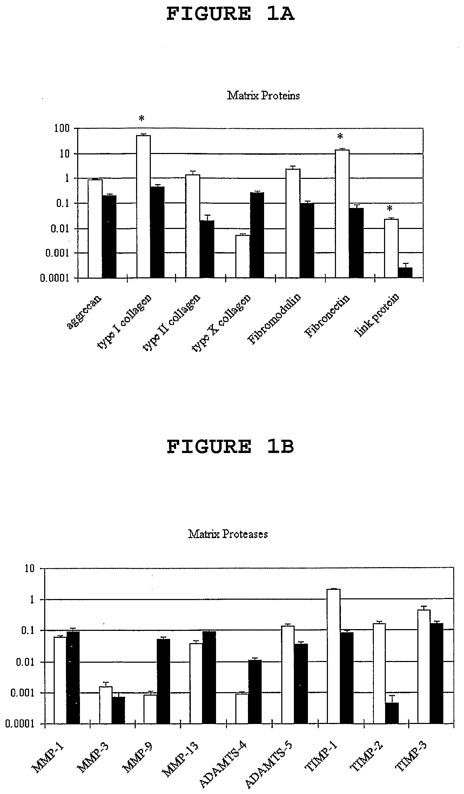

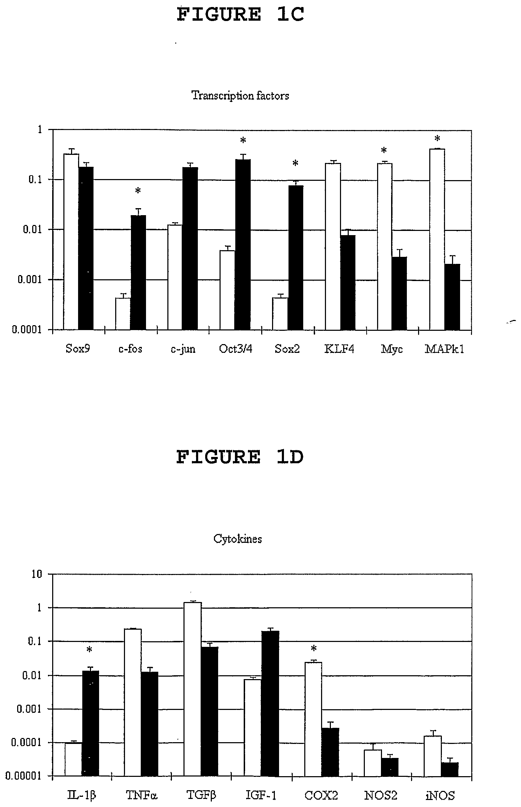

A 100g cartilage chip was excised from the non-weight bearing area of joint and placed into serum-free nutrient media. Each biopsy containing about 100 to 200 thousand cells, was expanded *in vitro* to approximately 10 million cells by the method described in the patent (PCT/AU2007/000362 entitled "Tenocyte Culture Method" ascribed to Zheng). After acceptable cell density was achieved cells were reconstituted into patients' own serum in a sealed glass vessel and transported to a site for implantation. At the arrival in the operating theatre, cells are re-heated to 37°C and injected onto the surface of a scaffold using a 23 gauge needle. A typical scaffold used was as described supra consisting of a collagen with/without polylysine coating. After the injection of cells onto the scaffold, the cells were spread onto the scaffold and allowed to incubate for not more than 2 hours before implantation. The controlled time for cell spreading allowed cells to attach, but not anchor into the scaffold thereby enabling rapid migration of the cells into the cartilage defect area after the cell-seeded scaffold was implanted.
As shown in Figure 1, the expression of a number of genes in human cells grown with (dark bars) or without (light bars) a collagen scaffold are significantly different (* = p<0.05). In particular, cells grown with the scaffold produce less type I and type II collagen and more MMP-1, MMP-9, MMP-13, ADAMTS-4, IL-1, c-fos, c-jun, Oct3/4 and Sox2, which are indicators of apoptosis. These results show that cells cultured on an implantable support are less viable that cells cultured without an implantable support.

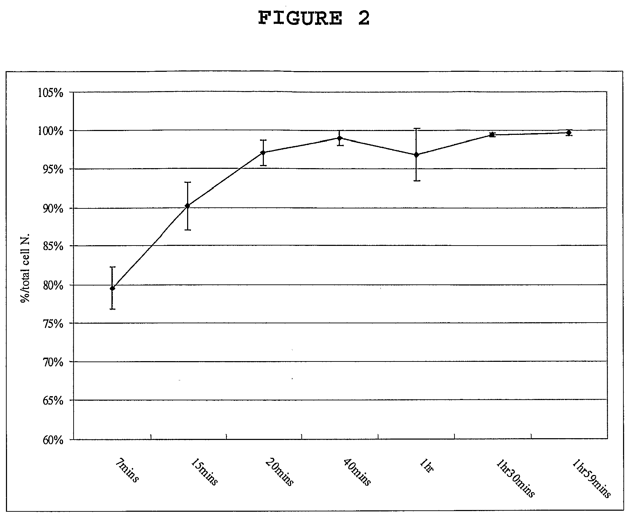

Figure 2 shows that significant numbers of cells adhere to the scaffold within 7 minutes of coming in contact with the scaffold. Adhesion of 100% of cells to the scaffold may be achieved within 40 minutes. At 20 minutes, 90% of cells are adhered to the scaffold. Accordingly, these results show that high levels of adherence can be achieved by contacting cells with an implantable support less than 2 hours before implantation. However, these results also indicate that cells should be contacted with an implantable support at least about 7 minutes before implantation to allow sufficient numbers of cells to adhere to the scaffold.

## Claims

1. A method of producing an implantable matrix comprising the steps of:
(a) providing an implantable support comprising cross-linked or uncross-linked collagen, and a sample of autologous mammalian chondrocyte cells; and
(b) applying said sample of autologous mammalian chondrocyte cells to the surface of said support to produce said implantable matrix;
wherein the cells are allowed to incubate for between about 20 minutes to 1 hour 20 minutes before implantation of the matrix; wherein said implantable support comprises a membrane.

2. A method according to claim 1, wherein the mammalian chondrocyte cells are human chondrocyte cells.

3. A method according to claim 1 or claim 2, wherein the mammalian chondrocyte cells are allowed to incubate for about 40 minutes before implantation of the matrix.

4. A method according to any one of claims 1 to 3, further comprising the step of coating the implantable matrix after the mammalian chondrocyte cells have adhered with a cell sealant prior to implantation.

5. A method according to claim 4, wherein the cell sealant is a fibrin sealant.

6. A method according to any one of claims 1 to 5, wherein the implantable support is heated to between 35°C and 37°C before the mammalian chondrocyte cells are applied.

7. A method according to any one of claims 1 to 6, wherein the mammalian chondrocyte cells are suspended in the subjects own serum before being applied to the implantable support.

## Patentansprüche

1. Ein Verfahren zum Herstellen einer implantierbaren Matrix, umfassend die Schritte:
(a) Bereitstellen eines implantierbaren Trägers, der vernetztes oder unvernetztes Kollagen enthält, und einer Probe autologer Säugetier-Chondrozytenzellen und
(b) Aufbringen der Probe autologer Säugetier-Chondrozytenzellen auf die Oberfläche des Trägers, um die implantierbare Matrix herzustellen,
wobei es den Zellen ermöglicht wird, vor der Implantierung der Matrix zwischen ungefähr 20 Minuten bis 1 Stunde 20 Minuten zu inkubieren, wobei der implantierbare Träger eine Membran umfasst.

2. Ein Verfahren gemäß Anspruch 1, wobei die Säugetier-Chondrozytenzellen menschliche Chondrozytenzellen sind.

3. Ein Verfahren gemäß Anspruch 1 oder Anspruch 2, wobei es den Säugetier-Chondrozytenzellen vor der Implantierung der Matrix gestattet wird, ungefähr 40 Minuten zu inkubieren.

4. Ein Verfahren gemäß einem der Ansprüche 1 bis 3, außerdem umfassend den Schritt des Beschichtens der implantierbaren Matrix, nachdem sich die Säugetierchondrozytenzellen angelagert haben, mit einem Zellabdichtmittel vor der Implantierung.

5. Ein Verfahren gemäß Anspruch 4, wobei das Zellabdichtmittel ein Fibrinabdichtmittel ist

6. Ein Verfahren gemäß einem der Ansprüche 1 bis 5, wobei der implantierbare Träger auf zwischen 35°C und 37°C erwärmt wird, bevor die Säugetierchondrozytenzellen aufgebracht werden.

7. Ein Verfahren gemäß einem der Ansprüche 1 bis 6, wobei die Säugetierchondrozytenzellen, bevor sie auf den implantierbaren Träger aufgebracht werden, im eigenen Serum eines Patienten suspendiert werden.

## Revendications

1. Procédé de production d'une matrice implantable comprenant les étapes :
(a) de fourniture d'un support implantable comprenant du collagène réticulé ou non réticulé, et d'un échantillon de cellules chondrocytes autologues de mammifère ; et
(b) d'application dudit échantillon de cellules chondrocytes autologues de mammifère à la surface dudit support pour produire ladite matrice implantable ;
dans lequel les cellules sont mises à incuber pendant entre environ 20 minutes et 1 heure 20 minutes avant l'implantation de la matrice ; dans lequel ledit support implantable comprend une membrane.

2. Procédé selon la revendication 1, dans lequel les cellules chondrocytes de mammifère sont des cellules chondrocytes humaines.

3. Procédé selon la revendication 1 ou la revendication 2, dans lequel les cellules chondrocytes de mammifère sont mises à incuber pendant environ 40 minutes avant l'implantation de la matrice.

4. Procédé selon l'une quelconque des revendications 1 à 3, comprenant en outre l'étape de revêtement de la matrice implantable après avoir mis à adhérer les cellules chondrocytes de mammifère avec une colle de cellule avant l'implantation.

5. Procédé selon la revendication 4, dans lequel la colle de cellule est une colle de fibrine.

6. Procédé selon l'une quelconque des revendications 1 à 5, dans lequel le support implantable est chauffé entre 35 °C et 37 °C avant l'application des cellules chondrocytes de mammifère.

7. Procédé selon l'une quelconque des revendications 1 à 6, dans lequel les cellules chondrocytes de mammifère sont mises en suspension dans le sérum propre aux sujets avant d'être appliquées au support implantable.
